# EUROPEAN PATENT APPLICATION

(11) **EP 4 806 478 A1**
(43) Date of publication of application: **16.09.2026**
(21) Application number: 25162616.4
(22) Date of filing: 10.03.2025
(51) Int. Cl.: A61M 5/20

(54) **ADD-ON MONITORING MODULE FOR A DELIVERY DEVICE**

(71) Applicant: Ypsomed AG, 3401 Burgdorf (CH)
(72) Inventor: Lindegger, Stefan, 4950 Huttwil (CH)
(74) Representative: Kleiner, Stefan

(57) **Abstract**

The invention relates to a drug delivery system comprising a drug delivery device (1) for dispensing a liquid drug through a needle. The delivery device (1) comprises a delivery device housing (2), a dose or dispensing member (3) movable relative to the device housing during dose setting or dose dispensing and a first conductive member (21) coupled to the dose or dispensing member (3). The delivery system further comprises an add-on module (30) for monitoring a drug delivery process with the drug delivery device (1). The add-on module (30) comprises a module housing (39) releasably attachable to the delivery device housing (2) and a first capacitive sensor (31) configured to sense a rotation of the first conductive member (21) relative to the device housing (2) based on a capacitive coupling between the first capacitive sensor (31) and the first conductive member (21).

## Description

### FIELD OF THE INVENTION

The present invention relates to a drug delivery device for injecting, delivering, administering, infusing or dispensing substances and/or liquids such as insulin, hormone preparations or vaccines. A drug delivery system comprises the drug delivery device with a dose or dispensing member movable relative to a device housing during dose setting or dose dispensing. The system comprises further an add-on module releasably attachable to the device housing and for monitoring a drug delivery process with the drug delivery device

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remain in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device-based therapies generally benefit from an electronic module or electronic unit embedded or integrated in the delivery device, or being part of an auxiliary, supplemental or electronic module or add-on module releasably attachable to the delivery device. The electronic module or add-on module monitors a drug delivery process, in order to proactively prevent false handling of the device and/or to keep track of the doses already applied, and generates data related to an instantaneous condition and/or use of the delivery device.

The prior art has described a variety of sensing concepts to monitor the drug delivery process. US 11541186 B2 discloses a module attachable to a drug delivery device. The module includes a capacitive sensor. A metal band including multiple discrete metal elements is mounted to a skirt of a dose dial member of the drug delivery device. The system further includes antennas mounted to a side wall opposite the metal band. A capacitive coupling is generated between each pair of antennas during rotation. The metal band has a shape which generates a varying signal upon rotation of the dose dial member skirt relative to the antennas and thus rotation of the dose dial member can be sensed by the module.

US 10850041 B2 discloses an injection pen with a housing and a stationary capacitive sensor assembly on a flexible sheet, to detect a rotating indicator element comprising a surface with a code pattern formed from conductive printed markings. The flexible sheet is mounted at least in part to the exterior of the pen housing.

### DESCRIPTION OF THE INVENTION

It is an objective of the present invention to provide a drug delivery monitoring system which has an enhanced reliability.

This objective is achieved by the drug delivery system, an add-on module and the method according to the independent claims. Preferred embodiments are evident from the dependent claims.

The invention relates to a drug delivery system comprising a drug delivery device for dispensing a liquid drug through an injection needle and an add-on module releasably attachable to the delivery device. The drug delivery device comprises:
- a delivery device housing defining a longitudinal axis;
- a dose or dispensing member movable relative to the device housing during dose setting or/and dose dispensing;
- a first conductive member directly or indirectly coupled to the dose or dispensing member such that a movement of the dose or dispensing member is transferred to the first conductive member.

The add-on module is releasably attachable to the device housing and comprises
- a module housing coaxially arranged to the longitudinal axis and extending along the longitudinal axis;
- a first sensor adapted to sense a rotation of the first conductive member relative to the device housing and relative to the module housing based on a capacitive coupling between the first sensor and the first conductive member. The first conductive member may have a rotational movement component and additionally a longitudinal movement component.

The add-on module comprises further a second capacitive sensor adapted to sense a movement along the longitudinal axis (or a longitudinal movement component) of the first conductive member or, if any, of a second conductive member different than the first conductive member. The second conductive member is preferably movable relative to the device housing and the module housing during dose setting or during dose dispensing. The second conductive member is preferably directly or indirectly coupled to the dose or dispensing member such that a movement of the dose or dispensing member is transferred to the second conductive member.

The add-on module includes a controller connected to the first and second sensor and wherein the controller is configured to determine an absolute position of the dose or dispensing member relative to the device housing and the module housing based on both a first signal from the first sensor (rotational movement information) and a second signal from the second sensor (longitudinal movement information).

The invention provides a capacitive sensing system with a first capacitive sensor to detect a rotational movement and a second capacitive sensor to detect a longitudinal movement of the dose or dispensing member.

According to prior art approaches usually a pattern provided by a conductive member for the rotational detection is repeated at least every 360°. That requires a counter to detect the rotations and to determine a relative total rotation of the dose or dispensing member. However, such an approach is prone to inaccuracies, in particular when contact is lost, or an interruption of the power supply occurs.

According to the invention the add-on module comprises a second capacitive sensor adapted to sense and to determine a position of the first or, if any, a second conductive member and thus of the dose and dispensing member with respect to the longitudinal axis. The first capacitive sensor is configured to sense and determine a position of the first and/or second conductive member preferably exclusively in the rotational direction around the longitudinal axis and preferably in a plane perpendicular to the longitudinal axis.

The second capacitive sensor is configured to sense and determine the position of the first or/and second conductive member preferably exclusively in the longitudinal axis.

That means the controller receives the rotational position information via first sensor signal from the first sensor and the longitudinal position information via second sensor signal from the second sensor. That allows to determine not only a relative movement of the dose or dispensing member but allows to reliably determine an absolute position of the dose or dispensing member relative to the device housing. That means due to the absolute longitudinal position information in combination with the rotational position information the controller is able to determine the absolute position of the dose or dispensing member relative to the device housing (and module housing).

The drug delivery device may be a disposable injection pen comprising a reservoir (cartridge) with the liquid drug, a semi-disposable injection device with a reusable drive unit and a replaceable receptacle including a reservoir, a reusable injection device with replaceable reservoir (cartridge) or an autoinjector comprising a syringe with a needle permanently attached to a syringe body. The drug delivery device preferably includes a plunger rod to dispense the drug from the reservoir.

The term "injection device", "injection pen" or "injector" refers to a device that is removed from the injection site after each medication event or drug delivery process, whereas the term "infusion system" or "infusion device" refers to a device with a cannula or needle that remains in the skin of the patient for a prolonged period of time, for example, several hours.

The dose or dispensing member may be a component of the dose and dispensing mechanism which is moved relative to the housing during dose setting or/and during dose dispensing. By way of example, the dose or dispensing member may be a rotatable dose set member, for example a dose setting sleeve, a dose knob or a number sleeve that is rotated when a user sets a dose and/or during dose dispensing when the dose is administered. Further, the dose or dispensing member may be a drive member, a clutch, a nut or a piston rod rotating during dispensing of a set dose, in particular when a user presses a release button or actives the device to dispense and administer the previously set dose.

The dose or dispensing member is coupled to the first conductive member and optionally, if any, to the second conductive member. The coupling may be direct or indirect. The latter may be via one or more intermediate members. The coupling between the dose and dispensing member and the first and/or second conductive member may include a transmission in form of a gear (e.g. toothed wheels). Alternatively, the first and/or second conductive member may be directly coupled, for example attached, fixedly connected, for example, by a form-fit or force-fit engagement to the dose or dispensing member.

**In** all embodiments, a movement or position of the first and optionally the second conductive member are indicative of a position or movement of the dose or dispensing member.

The drug delivery device comprises at least one first conductive member. Optionally, the delivery device may include a second conductive member. The first and, if any, the second conductive member are electroconductive and electrodes. The conductive member may be, for example, a metal plate, a wire, a conductive coating or any element which is made of an electroconductive material.

The first and second capacitive sensors may each include at least one active electrode providing an electric field such that a capacitive coupling is generated between the sensor electrode and the first conductive member (first electrode) and, if any, the second conductive member (second electrode). The sensors are adapted to measure a capacitance or/and change in capacitance between the sensor electrode and the electrode of the first conductive member and optionally between the sensor electrode and the electrode of the second conductive member.

The first conductive member may include a pattern of recurring elements, for example, rectangles circumferentially arranged. Additionally, the first conductive member or, if any, the second conductive member may have an element extending along the longitudinal axis which changes its shape along the longitudinal axis, for example, a triangular or a form or shape that is non-recurring along the longitudinal axis to allow an absolute position determination based on a sensor signal that is unique for every possible position along the longitudinal axis.

The controller in the add-on module is configured to measure the strength or/and a change of the capacitive coupling between the sensor electrodes and the movable electrodes of the conductive member. Accordingly, the controller can determine the presence or a movement of the first conductive and/or second conductive member when the conductive member interacts with the electric filed and thus changes the capacitive coupling.

The first capacitive sensor is preferably exclusively configured to sense and detect a rotational movement or position circumferentially around the longitudinal axis and is preferably not configured to detect a position or movement along longitudinal axis. The second capacitive sensor is preferably exclusively configured to sense and detect a longitudinal movement along the longitudinal axis and is not configured to detect a rotational position or movement. The sensing direction is determined by the shape of the conductive member and the location and alignment of the capacitive sensor relative to the first and, if any, second conductive member.

The controller is configured to combine the sensor signals from the first and second sensor to determine the absolute position of the dose and dispensing member.

The first and second capacitive sensor are preferably contactless sensors and thus any mechanical wear between moving components can be avoided.

The controller is preferably arranged inside the add-on module housing, for example, in an electronic module. The controller may be a microcontroller or FPGA or any other signal processing unit.

The add-on module is a supplemental module that is releasably attachable to the complete delivery device. That means the add-on module is not part and not a component of the delivery device itself. The drug delivery device can work without and independent from the add-on module.

The add-on module may include connecting means such as, for example, clamps or snap-fit elements to releasably attach the add-on module to the housing of the drug delivery device.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances
The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment, the first capacitive sensor includes two pairs of oppositely arranged rotational sensor electrodes. The pairs are preferably arranged around the longitudinal axis. The first conductive member includes preferably a pair of rotatable electrodes arranged radially inside the two pairs of sensor electrodes. That means in a preferred arrangement the electrode pair of the conductive member is on an inner circle where the two electrodes are oppositely arranged to each other. The two pairs of sensor electrodes are arranged on an outer circle such that the electrode pair of the conductive member is radially inside the sensor electrodes.

Two electrodes (sensor or conductive member) arranged oppositely to each other allow to compensate for position tolerances, mechanical play and variations during rotation of the conductive member relative to the sensor. That means in comparison with a one electrode approach the two oppositely arranged electrodes may reduce the influence of tolerances and inaccuracies.

The conductive member and the first and second capacitive sensor may each include more than one pair of electrodes, for example, three or four pairs of electrodes.

The second capacitive sensor includes preferably two longitudinal sensor electrodes spaced apart from each other along the longitudinal axis. This allows for a reliable sensing of an electrode of the conductive member and thus of the dose or dispensing member with respect to the longitudinal axis. The two longitudinal sensor electrodes are further arranged opposite each other with respect to the circumference of the longitudinal axis. That means the longitudinal sensor electrodes are about 180° spaced apart from each other in a circumferential direction.

In a preferred embodiment the first conductive member and/or the second conductive member is a metal plate or metal sheet. The metal plate or sheet is electroconductive and allows capacitive coupling with sensor electrodes. Furthermore, the plate or sheet may be attached or fixed to an outer surface of the dose or dispensing member or to an intermediate member coupled to the dose or dispensing member.

The metal plate or sheet allows a compact design of the delivery device as the plate or sheet attached to an outer surface of a movable member needs only minimal extra space and thus does not require complete redesign of an existing dose and dispensing mechanism.

As an alternative to the above, the first or/and second conductive member may be implemented in form of a conductive coating or a conductive ink. The coating or the ink may comprise metal particles allowing a capacitive coupling with the sensor electrode. The coating or ink may be applied to an outer surface of the dose or dispensing member or an intermediate member coupled to the dose and dispensing member. Similarly to the above, the coating or ink does not require extra space and thus contributes to a compact delivery device design.

The first conductive member preferably comprises a row of rectangular-shaped elements circumferentially arranged around the longitudinal axis and connected to each other. The elements allow for a reliable detection of a rotation based on a change in the capacitance caused by the rotating rectangular-shaped elements relative to a sensor electrode.

Preferably, the delivery device includes the second conductive member which may include a longitudinally extending element. The shape of the element varies along the longitudinal axis such that the capacitance alters during a movement along the longitudinal axis relative to a sensor electrode, in particular a surface area of the longitudinally extending element may decrease along the longitudinal axis. Preferably, the longitudinally extending element may be helically arranged around the longitudinal axis. For example, the longitudinally extending element may be a stripe or band extending in screw-like form around the longitudinal axis wherein the strip or band becomes smaller along the longitudinal axis. This allows the second sensor to sense a position of the second conductive member and thus of the dose or dispensing member along the longitudinal axis.

Therefore, in this embodiment the first capacitive sensor may be capacitively coupled to the first conductive member, preferably to rectangular-shaped elements, for sensing an exclusive rotational movement and the second capacitive sensor may be capacitively coupled to the second conductive member, preferably to the longitudinally extending element, for sensing an exclusive longitudinal movement.

The second conductive member may be electrically connected to the first conductive member.

In a preferred embodiment the first and second conductive member are connected to each other or integrally formed in a common plate, in particular in a metal plate or metal sheet. That allows an easy and cost-effective production of the conductive members.

Alternatively, the first and second conductive members may be separated from each other and electrically isolated. In particular the first and second conductive member may be arranged on two distinct components. That may separate the capacitive couplings of the two sensors and may thus help to avoid mutual interference.

Preferably, the dose or dispensing member is a number sleeve rotatable around the longitudinal axis and comprising visible markings representing a dose value or an amount of medication to be dispensed. The number sleeve is preferably rotatable relative to the housing during both dose setting and dose dispensing.

The first conductive member may be rotationally and axially fixed to the number sleeve and may extend in a circumferential direction for rotation sensing and the second conductive member may extend along the longitudinal axis for longitudinal sensing.

The controller may determine an absolute position of the number sleeve relative to the housing based on the first sensor signal (rotation) and second sensor signal (longitudinal movement). As the number sleeve rotates during both, dose setting and dose dispensing the set dose as well the actually dispensed dose can be determined and thus a reliable monitoring of the whole injection process is enabled.

The first and, if any, the second conductive member may be connected to the number sleeve, preferably on an outer surface, for example, in form of a metal layer or sheet or conductive coating or ink or integrated in the sleeve, for example, by conductive particles molded integrally in a plastic number sleeve.

In a further embodiment the drug delivery device includes a third conductive member coupled or fixed to a trigger member and movable along the longitudinal axis. In this case, the add-on module preferably comprises a third capacitive sensor configured to sense an exclusive longitudinal movement of third conductive member. The trigger member may be a component of the dose and dispensing mechanism which component exclusively moves along the longitudinal axis without rotation.

In a preferred embodiment the trigger member is a push button or release button to start an injection. The button is in a first axial position during dose setting and in a second axial position during dose dispensing. That means the third conductive member preferably moves exclusively along the longitudinal when the button is pressed or released.

The actuation of the button may be detected and an current state of the drug delivery device can be determined of the signals of each of the first, second and third capacitive sensor.

The delivery device is preferably a pen-shaped injection device including a dose and dispensing mechanism. In a preferred embodiment the dose or dispensing member is a number sleeve indicating a dose and rotating during both dose setting and dose dispensing. Preferably, the dose and dispensing mechanism includes a dose knob, the number sleeve a clutch member and a drive member to move a piston rod in dispensing direction for dispensing the drug. The clutch member may be coupled to the dose knob and decoupled from the drive member when the button is in the first axial position and the clutch member is decoupled from the dose knob and coupled to the drive member when the button is in the second axial position.

Preferably, the add-on module has a housing with an opening adapted to accommodate the device housing for releasable attachment of the add-on module to the drug delivery device. That means the add-on module housing may at least partially enclose or cover the delivery device housing, particularly a pen-shaped device housing. That means in a plane perpendicular to the longitudinal axis the add-on module housing envelops the device housing at least a half of a circumference of the device housing.

Such an add-on module housing allows a reliable coupling of the add-on housing with the device housing.

The invention relates further to the add-on module for monitoring a drug delivery with the drug delivery device, the add-on module comprising
- a module housing releasably attachable to a drug delivery device housing
- a first capacitive sensor adapted to sense a rotation of a first conductive member of the delivery device in an attached state relative to the module housing based on a capacitive coupling;
That the add-on module comprises further a second capacitive sensor adapted to sense a movement exclusively along the longitudinal axis of the first conductive member or of a second conductive member of the delivery device and movable relative to the module housing during dose setting or dose dispensing

The add-on module includes a controller connected to the first and second sensor and adapted to determine the absolute position of a dose or dispensing member relative to the module housing based on both a first signal from the first sensor and a second signal from the second sensor.

The invention further relates to a method for determining an absolute position of a dose or dispensing member relative to a drug delivery device housing, the method comprising the steps of
- moving the dose or dispensing member relative to the device housing to set a dose or to dispense a dose;
- sensing, by a first capacitive sensor of an add-on module attached to the delivery device, a rotation of a first conductive member directly or indirectly coupled to the dose or dispensing member and based on a capacitive coupling;
- sensing, by a second capacitive sensor of the add-on module, a movement along the longitudinal axis of the first or a second conductive member;
- processing, by a controller of the add-on module, a first sensor signal from the first capacitive sensor and a second signal of the second capacitive sensor;
- determining, by the controller, an absolute position of the dose or dispensing member relative to the device housing based on both the first and second sensor signal.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a sectional view of an injection pen with an attached add-on module according to the invention;
- Fig. 2: depicts schematic view of the first and second conductive member and the sensors of the add-on module and
- Fig. 3: depicts schematic and sectional view of the electrodes and sensors in a plane perpendicular to the longitudinal axis.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

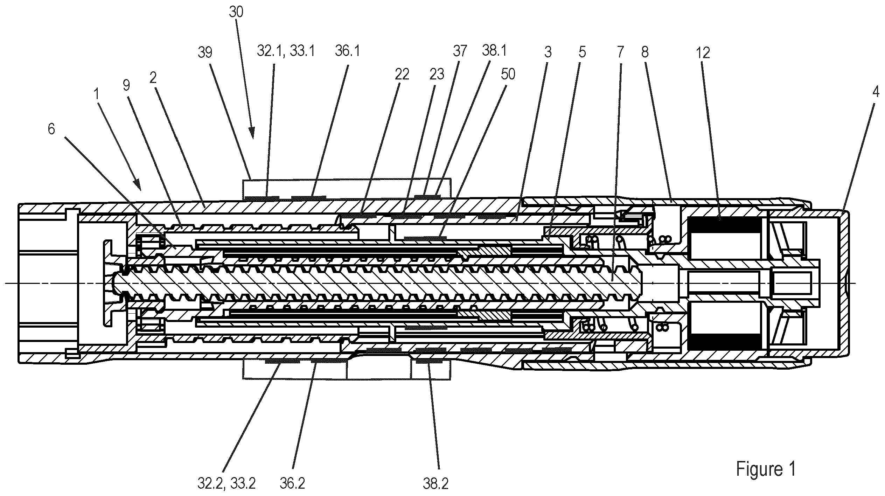

Figure 1 depicts a drug delivery device in the form of a reusable spring-driven injection pen 1. Figure 1 depicts a sectional view where the cut for the section runs along the longitudinal axis of the pen.

The injection pen 1 includes a housing 2 accommodating a dose and dispensing mechanism. The injection pen further includes a cartridge holder, a cartridge with the liquid drug and an injection needle mounted on a distal end (all not shown). The cartridge holder can be attached or detached from housing to replace a cartridge as known in the art.

Furthermore, figure 1 schematically depicts an add-on module 30 releasably attached to an injection pen housing 2 of the injection pen 1. The add-on module 30 includes a housing 39 with an opening accommodating the injection pen 1 such that the add-on module housing at least partially envelopes the injection pen housing 2.

The pen includes a sleeve-shaped insert 9 axially and rotationally fixed to the housing 2 or integrally formed with the housing. The insert 9 has on its outside a thread engaging an inner thread of an indication barrel or number sleeve 3 (also named as scale drum) which is located coaxially inside the housing 2. The number sleeve 3 includes numbers indicating a dose values which can be seen by the user through an opening in the housing 2.

The injection pen includes a sleeve-shaped insert fixed to the housing or integrally formed with the housing. The insert includes an outer thread engaging an inner thread or thread segment of a dose member or number sleeve 3 which is located coaxially inside the housing 2. The number sleeve 3 indicates dose values which a user can see through an opening or window in the housing.

The injection pen 1 further includes a clutch sleeve 5 (or dispensing member), a drive sleeve 6, a dose knob 8, a drive spring 12 and a button 4 that can be pressed in distal direction by the user to start the injection. The button 4 is adapted to selectively couple the clutch sleeve 5 to the dose knob 8 or to couple the clutch sleeve 5 to the drive sleeve 6 for dispensing a dose as will be described in detail below.

The clutch sleeve 5 is coaxially arranged inside the number sleeve 3 and the drive sleeve 6 in turn is located inside the clutch sleeve 5. The clutch sleeve 5 can be selectively, releasably and rotationally coupled to the drive sleeve 6 by a spline engagement. The drive sleeve 6 is permanently rotationally coupled to the plunger rod 7 which is in threaded engagement with the housing 2.

To set a dose the user rotates the dose knob 8 and as the button 4 is in a proximal position the clutch sleeve is rotationally coupled to the dose knob 8 but the clutch sleeve 5 is decoupled from the drive sleeve 6. Rotating the dose knob 8 rotates thus the clutch sleeve 5 and also the number sleeve 3 and additionally biases the drive spring 12. A one-way ratchet prevents unintentional release of the biased spring 12.

To dispense the dose the user presses the button 4 in distal direction. That triggers several operations. First, the clutch sleeve 5 is thus moved distally along the longitudinal direction and thereby the clutch sleeve 5 gets into engagement with the drive sleeve and is thus rotationally coupled with the drive sleeve 6. Second, the axial movement of the clutch sleeve 5 causes the dose knob 8 to be decoupled from the clutch sleeve 5. The number sleeve 3 remains permanently coupled to the clutch sleeve 5. Third, the clutch sleeve 5 is not long coupled to the one-way ratchet meaning that it is free to rotate. That means the biased spring 12 can rotate the clutch sleeve 5, the drive sleeve 6 and thus the plunger rod 7 which is screwed in distal direction and dispenses the set dose.

As the number sleeve 3 remains rotationally coupled to the clutch sleeve 5 the number sleeve 3 is rotated during both dose setting and dose dispensing.

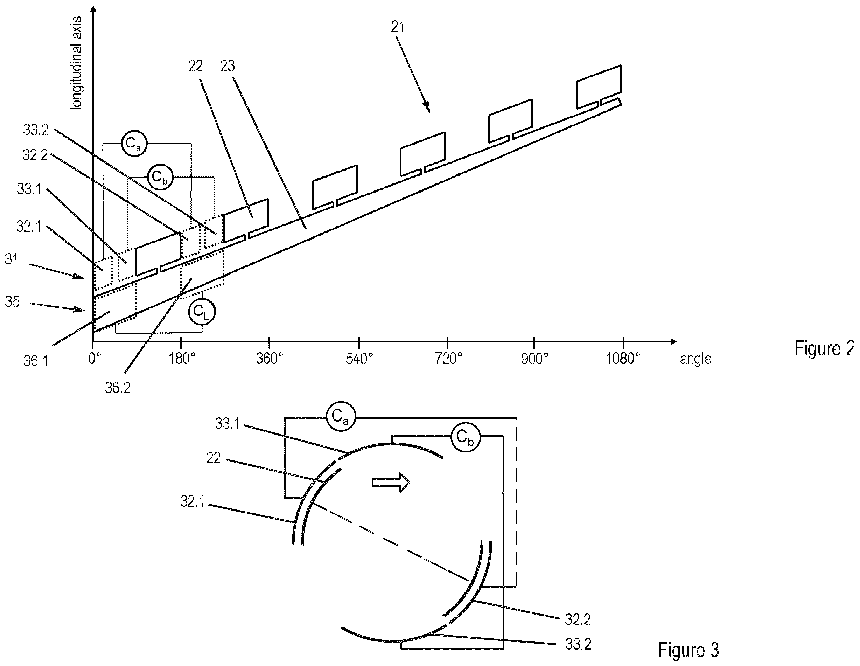

As depicted in figure 1 and as described in detail in the following the number sleeve 3 comprises a first and second conductive member in the form of a metal electrode plate 21 on its outer surface. The add-on module 30 includes two capacitive sensors 31, 35 (figure 2) that can sense the rotational and longitudinal movement and position of the number sleeve 3 based on a capacitive coupling between sensor electrodes and the electrode plate 21 on the number sleeve 3.

Figure 2 depicts a graph having a horizontal axis representing the circumference of the number sleeve from 0 to 1080° and a vertical axis representing the longitudinal axis of the number sleeve from 0 to a total length L. Figure 2 depicts a developed view of the electrode plate 21. That means the electrode plate 21 is depicted in a plane (one dimension) for a better view instead of a view showing the electrode plate 21 in an assembled and wrapped state on the number sleeve 3.

As can be seen in figure 2 the electrode plate 21 comprises a plurality of consecutive rectangular-shaped elements 22 (first conductive member) that are connected to each other by a thin bar or strip. The electrode plate further includes a strip 23 (second conductive member) extending along the length L with a width that decreases along the length.

When assembled and attached to an outer surface of the number sleeve 3 the rectangular-shaped elements 22 are arranged around a circumference of the number sleeve. The strip 23 is attached in a helical manner when the electrode plate 21 is assembled to the number sleeve.

The rectangular-shaped elements 22 are adapted to be sensed by a first rotational capacitive sensor 31 of the attached add-on module to detect a rotational movement. As the number sleeve 3 rotates during dose setting as well as during dose dispensing the capacitance changes as one rectangular-shaped element 22 passes one after the other the sensor electrode and hence the controller can determine a rotational movement and position of the number sleeve 3.

Stripe 23 is adapted to be sensed by a second longitudinal capacitive sensor 35 to detect a movement along the longitudinal axis of the number sleeve 3. As the number sleeve 3 moves with a screw-movement and thus has a longitudinal movement component and as the strip 23 alters its form along the longitudinal axis a change in capacitance can be measured during dose setting and dose dispensing and hence the controller is configured to determine a longitudinal movement and position of the number sleeve 3.

The add-on module 30 includes a controller adapted to process a first sensor signal from the first capacitive sensor 31 and a second signal from the second capacitive sensor 35. The controller is further configured to determine an absolute position of the number sleeve 3 relative to the drug delivery device housing 2 (and hence relative to the add-on module housing 39) based on both the first and second sensor signal.

Referring to figure 3, it can be seen that the rectangular-shaped elements 22 are attached to the number sleeve 3 such that in a plane perpendicular to the longitudinal axis two elements 22 are located opposite each other. This arrangement allows to compensate for mechanical play and tolerances because the deviation in the distance between the electrode elements and the sensor electrodes compensate each other.

As mentioned above, the add-on module 30 includes a rotational capacitive sensor 31 and a longitudinal capacitive sensor 35. In the embodiment shown in figure 2 and 3 the rotational capacitive sensor 31 includes two sensor channels each channel including a pair of sensor electrodes 32.1, 32.2, 33.1, 33.2 forming in total two capacitances Ca, Cb. The sensor electrodes of each pair are arranged opposite each other as shown in figure 3.

The capacitances Ca, Cb include a capacitive coupling between the sensor electrodes 32.1, 32.2, 33.1, 33.2 and the rotatable rectangular-shaped elements 22 to sense a rotational movement component of the number sleeve 3. As shown in figure 2 the longitudinal capacitive sensor 35 includes two sensor electrodes 36.1, 36.2 separated from each other and arranged along the longitudinal axis and having a capacitance CL. That capacitance includes a capacitive coupling between the sensor electrodes 36.1, 36.2 and the strip element 23 to sense a longitudinal movement component of the number sleeve as described above.

The excitation of the channels may be alternating to avoid any unwanted interference. A sample frequency per channel may be around 50Hz but may also be higher up to 400Hz.

In a further embodiment the sensor may have three sensor electrodes or the channels may be duplicated or even tripled and hence the rotational sensor may include four or more pairs of sensor electrodes.

The controller is configured to process both the rotational sensor signal of the rotational sensor 31 with the capacitances Ca, Cb and the longitudinal sensor signal of the longitudinal sensor 35 with the capacitance CL and the absolute position of the number sleeve can be determined.

Namely, based on the sensor signals the controller is configured to determine a dose which is dialed by the user based on a number sleeve movement. Additionally, the controller can determine a dose which is actually dispensed based on a number sleeve movement in the counter (dispensing) direction. The detection of the number sleeve movement may be used to activate or wake up electronics which are in an inactive or sleep mode during idle time.

The detected dialed dose or dispensed dose may be communicated to the user, for example via a display of the add-on module or via transmission from the add-on module to an external device such as a remote computer, cloud server or user mobile device.

In addition, the drug delivery system can include an axial button sensor configured to detect an axial movement of the button or clutch sleeve and thus can detect a current injection state. As shown in figure 1 the injection pen includes an axial electrode 50 on the clutch sleeve 5 which moves axially when the clutch sleeve is moved with the button 4 between a dose setting position and a dose dispensing position. Alternatively, the axial electrode may be arranged on the button.

Accordingly, the add-on module 30 further includes a third capacitive sensor 37 (figure 1) having two oppositely arranged sensor electrodes 38.1, 38.2 (figure 1) configured to establish a capacitive coupling to the axial electrode 50. The controller in the add-on module is configured to process the third sensor signal from the third sensor to determine an axial position of the clutch sleeve and the button. Hence, the controller can determine whether the injection pen is in a dose setting state (clutch sleeve or button in proximal position) or in a dose dispensing state (clutch sleeve or button distal position). Furthermore, the controller can determine a holding time during which the user holds the injection pen onto the injection site right after the injection.

As stated above, the determined state may be communicated to the user either by a display of the add-on module or by an external device.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | |
|---|---|---|---|
| 1 | Injection pen | 37 | third sensor |
| 2 | housing | 38.1 | sensor electrode |
| 3 | number sleeve | 38.2 | sensor electrode |
| 4 | button | 39 | module housing |
| 5 | clutch sleeve | | |
| 6 | drive sleeve | 50 | axial electrode |
| 7 | plunger rod | | |
| 8 | dose knob | | |
| 9 | insert | | |
| 21 | electrode plate | | |
| 22 | rectangular-shaped elements | | |
| 23 | strip element | | |
| 30 | add-on module | | |
| 31 | rotational sensor | | |
| 32.1 | sensor electrode | | |
| 32.2 | sensor electrode | | |
| 33.1 | sensor electrode | | |
| 33.2 | sensor electrode | | |
| 35 | longitudinal sensor | | |
| 36.1 | sensor electrode | | |
| 36.2 | sensor electrode | | |

## Claims

1. Drug delivery system comprising a drug delivery device (1) for dispensing a liquid drug through a needle, the delivery device (1) comprising:
- a delivery device housing (2) defining a longitudinal axis;
- a dose or dispensing member (3) movable relative to the device housing during dose setting or dose dispensing;
- a first conductive member (21) directly or indirectly coupled to the dose or dispensing member (3) such that a movement of the dose or dispensing member (3) is transferred to the first conductive member (21);
the delivery system further comprising an add-on module (30) for monitoring a drug delivery process with the drug delivery device (1), the add-on module (30) comprising
- a module housing (39) releasably attachable to the delivery device housing (2);
- a first capacitive sensor (31) configured to sense a rotation of the first conductive member (21) relative to the device housing (2) based on a capacitive coupling between the first capacitive sensor (31) and the first conductive member (21),
**characterized in that** the add-on module (30) further comprises a second capacitive sensor (35) configured to sense a movement along the longitudinal axis of the first conductive member (21) or of a second conductive member directly or indirectly coupled to the dose or dispensing member (3) such that a movement of the dose or dispensing member is transferred to the second conductive member
wherein the add-on module (30) includes a controller adapted to determine an absolute position of the dose or dispensing member (3) relative to the device housing (2) based on both a first signal from the first capacitive sensor (31) and a second signal from the second capacitive sensor (35).

2. Drug delivery system according to claim 1, wherein the first capacitive sensor (31) includes two pairs of oppositely arranged sensor electrodes (31.1, 31.2) and the first conductive member (21) includes a pair of rotatable electrodes (22) arranged radially inside the two pairs of sensor electrodes (31.1, 31.2).

3. Drug delivery system according to claim 1 or 2, wherein the second capacitive sensor (35) includes two sensor electrodes (36.1, 36.2) spaced apart from each other along the longitudinal axis.

4. Drug delivery system according to any of claims 1 to 3, wherein the first or/and second conductive member (21) is a metal plate or metal sheet.

5. Drug delivery system according to any of claims 1 to 3, wherein the first or/and second conductive member (21) is a conductive coating or a conductive ink comprising metal particles.

6. Drug delivery system according to any of claims 1 to 5, wherein the first conductive member (21) comprises a row of rectangular-shaped elements (22) circumferentially arranged around the longitudinal axis and connected to each other.

7. Drug delivery system according to claim 6 wherein the second conductive member comprises a longitudinally extending element (23) wherein the shape of the element varies along the longitudinal axis such that the capacitance alters during a movement along the longitudinal axis.

8. Drug delivery system according to claim 7 wherein the first and second conductive member are connected to each other or integrally formed in a common plate.

9. Drug delivery system according to any of claims 1 to 8, wherein the dose or dispensing member is a number sleeve (3) comprising visible markings representing a dose value and wherein the number sleeve (3) is rotatable relative to the housing (2) during both dose setting and dose dispensing and wherein the first conductive member (21) is rotationally and axially fixed to the number sleeve (3).

10. Drug delivery system according to any of claims 1 to 9, wherein the drug delivery device (1) comprises a third conductive member (50) coupled to a trigger member (4) movable along the longitudinal axis and the add-on module (30) comprises a third capacitive sensor (37) configured to sense an exclusive longitudinal movement of third conductive member (50).

11. Drug delivery system according to claim 10, wherein the trigger member (4) is a button which is in a first axial position during dose setting and in a second axial position during dose dispensing.

12. Drug delivery system according to claim 11, wherein the delivery device is an injection device (1) including a dose knob (8), a clutch member (5) and a drive member (6) to move a piston rod in dispensing direction for dispensing the drug, wherein the clutch member (5) is coupled to the dose knob (8) and decoupled from the drive member when the button (4) is in the first axial position and the clutch member (5) is decoupled from the dose knob (8) and coupled to the drive member (6) when the button (4) is in the second axial position.

13. A drug delivery system according to any of claims 1 to 12, wherein the add-on module housing (39) has an opening adapted to accommodate the device housing (2) for releasable attachment.

14. An add-on module (30) for monitoring a drug delivery process with a drug delivery device (1), the add-on module comprising
- a module housing (39) releasably attachable to a drug delivery device housing (2);
- a first capacitive sensor (31) configured to sense a rotation of a first conductive member (21) of the delivery device in an attached state relative to the module housing based on a capacitive coupling;
**characterized in that** the add-on module (30) comprises further a second capacitive sensor (35) configured to sense a movement along the longitudinal axis of the first conductive member (21) or of a second conductive member of the delivery device (1) and movable relative to the module housing (39) during dose setting or dose dispensing and
wherein the add-on module (30) further includes a controller configured to determine an absolute position of a dose or dispensing member (3) relative to the module housing based on both a first signal from the first sensor and a second signal from the second sensor.

15. A method for determining an absolute position of a dose or dispensing member (3) of a drug delivery device (1) relative to a drug delivery device housing (2), the method comprising the steps of
- moving the dose or dispensing member (3) relative to the device housing (2) to set a dose or to dispense a dose;
- sensing, by a first capacitive sensor (31) of an add-on module (30) attached to the delivery device (1), a rotation of a first conductive member directly or indirectly coupled to the dose or dispensing member and based on a capacitive coupling;
- sensing, by a second capacitive sensor of the add-on module, a movement along the longitudinal axis of the first or a second conductive member;
- processing, by a controller of the add-on module, a first sensor signal from the first capacitive sensor and a second signal of the second capacitive sensor;
- determining, by the controller, an absolute position of the dose or dispensing member relative to the device housing based on both the first and second sensor signal.
